(19) Europäisches Patentamt
European Patent Office
Office européen des brevets

(11) **EP 4 088 711 A1**

(12) **EUROPÄISCHE PATENTANMELDUNG**

(43) Veröffentlichungstag:
**16.11.2022 Patentblatt 2022/46**

(21) Anmeldenummer: **22160692.4**

(22) Anmeldetag: **08.03.2022**

(51) Internationale Patentklassifikation (IPC):
*A61K 8/04* (2006.01)      *A61K 8/19* (2006.01)
*A61K 8/37* (2006.01)      *A61K 8/39* (2006.01)
*A61K 8/34* (2006.01)      *A61Q 15/00* (2006.01)

(52) Gemeinsame Patentklassifikation (CPC):
**A61Q 15/00; A61K 8/046; A61K 8/19; A61K 8/34;
A61K 8/375; A61K 8/39;** A61K 2800/412

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(30) Priorität: **04.05.2021 DE 102021111500**

(71) Anmelder: **Henkel AG & Co. KGaA
40589 Düsseldorf (DE)**

(72) Erfinder:
• **Doering, Thomas
41540 Dormagen (DE)**
• **Solich, Daniel
40764 Langenfeld (DE)**

(54) **SPRÜHBARE, KOSMETISCHE ZUSAMMENSETZUNG UMFASSEND POLYGLYCERINESTER**

(57)     Die vorliegende Erfindung betrifft eine sprühbare, kosmetische Zusammensetzung umfassend Polyglycerinester, ein kosmetisches Mittel umfassend diese Zusammensetzung und die Verwendung der Zusammensetzung als Deodorant.

**EP 4 088 711 A1**

**Beschreibung**

Die vorliegende Erfindung betrifft eine sprühbare, kosmetische Zusammensetzung umfassend Polyglycerinester, ein kosmetisches Mittel umfassend diese Zusammensetzung und die Verwendung der Zusammensetzung als Deodorant.

Die Verwendung von Körperpflegemitteln, insbesondere von Duftstoffen, zur Bekämpfung von unangenehmem Körpergeruch hat eine lange Tradition. Während im alten Ägypten und in der Antike hauptsächlich Parfümöle eingesetzt wurden, werden heute hauptsächlich Deodorants verwendet, die vorwiegend in den Achselhöhlen aufgebracht werden, um ungewolltem Schweißgeruch entgegenzuwirken. Deodorants sind in einer Reihe verschiedener Darreichungsformen verfügbar, beispielsweise als Spray, Pumpspray, Roller, Stift, getränkte Tücher, Creme, Puder oder Kristalle. Die Darreichung in Form eines Sprays erfolgt in der Regel mit Hilfe von Treibgasen, also Gasen, die komprimiert oder druckverflüssigt werden, um durch ihren Druck Flüssigkeiten oder Feststoffpulver zu fördern oder zu zerstäuben. In diesem Zusammenhang haben unter anderem die unter der Sammelbezeichnung FCKW bekannten Kohlenwasserstoffe traurige Berühmtheit erlangt.

Im Angesicht des steigenden Umweltbewusstseins besteht der Wunsch alternativen für die herkömmlichen Treibmittel zu finden und insbesondere inerte Gase wie Stickstoff oder komprimierte Luft als Treibmittel zu verwenden. Diese Systeme zeigen jedoch im Vergleich mit Systemen mit Flüssiggas eine schlechtere Aerosolbildung. So sind die im Sprühstrahl vorliegenden Tröpfchen bereits zu Beginn recht groß und wachsen bei Druckabfall weiter an. Dies resultiert für den Anwender in einem unerwünscht nassen Hautgefühl. Außerdem können größere Tropfen aus dem Sprühstrahl herausfallen, ein Effekt, der unter dem Stichwort "rainfall-Effekt" bekannt ist, und gelangen so gar nicht erst an ihren Zielort.

Zwar wurde versucht, durch Erhöhung des Drucks die Nachteile, die die inerten Gase als Treibmittel mit sich bringen, auszugleichen. Diese Maßnahme hat sich insofern jedoch nicht als praktikabel erwiesen, als das der Druck bei jeder Nutzung unweigerlich absinkt und darüber hinaus eine Drucksteigerung robustere Aeorosolbehälter erfordert, die wiederum mit einem höheren Energieaufwand in der Herstellung verbunden sind und so dem Ziel der Nachhaltigkeit entgegenstehen.

Es ist daher eine Aufgabe der vorliegenden Erfindung eine sprühbare Zusammensetzung zur Verfügung zu stellen, die die Nachteile des Stands der Technik überwindet.

Es wurde überraschend gefunden, dass durch den Zusatz von Polyglycerinestern die Tröpfchengröße im Sprühstrahl reduziert werden kann. Auch einem Anwachsen der Tröpfchengröße bei abfallendem Druck konnte so entgegengewirkt werden und ein über alle Anwendungen angenehmes Hautgefühl erreicht werden.

Daher ist ein erster Gegenstand der vorliegenden Erfindung eine sprühbare, kosmetische Zusammensetzung umfassend:

    a) Ethanol;
    b) Polyglycerinester;
    c) Wasser; und
    d) Treibmittel.

Als besonders vorteilhaft im Rahmen der vorliegenden Erfindung haben sich Polyglycerinester mit einem bestimmten HLB-Wert erwiesen. Der HLB-Wert (HLB: hydrophylic-lipophilic balance) beschreibt den hydrophilen und lipophilen Anteil von Molekülen und kann beispielsweise mittels der Methode nach Griffin wie folgt bestimmt werden:

$$HLB = 20 \times \left(1 - \frac{M_l}{M}\right)$$

wobei $M_l$ die Molmasse des lipophilen Anteils des Moleküls ist und M die Molmasse des gesamten Moleküls und der Faktor 20 ein festgelegter Skalierungsfaktor ist.

Im Rahmen der vorliegenden Erfindung sind insbesondere solche Polyglycerinester bevorzugt, die einen HBL-Wert von weniger als 15 haben. Daher ist eine Ausführungsform der erfindungsgemäßen Zusammensetzung bevorzugt, in der der Polyglycerinester einen HBL-Wert von 9 bis 15, vorzugsweise 11 bis 14 aufweist, bestimmt gemäß Molekulargewichtsberechnung nach Griffin.

Die vorliegende Erfindung hat das Ziel, eine sprühbare Zusammensetzung zur Verfügung zu stellen, die bei besserer Umweltverträglichkeit die gleichen Eigenschaften bezüglich Sprühbarkeit und Tröpfchengröße aufweist, wie herkömmliche Zusammensetzungen. In einer bevorzugten Ausführungsform der erfindungsgemäßen Zusammensetzung verwendet diese als Treibmittel daher ein komprimiertes Gas, das vorzugsweise ausgewählt ist aus der Gruppe bestehend aus Luft, Stickstoffoxid, Stickstoff, Kohlenstoffdioxid und Mischungen hiervon. In einer besonders bevorzugten Ausführungsform wird Stickstoff als Treibmittel verwendet.

Der Anteil an Polyglycerinester in der erfindungsgemäßen Zusammensetzung beträgt vorzugsweise 0,05 bis 5 Gew.-%, besonders bevorzugt 0,2 bis 0,6 Gew.-%, jeweils bezogen auf das Gesamtgewicht der Komponenten a) bis c) der erfindungsgemäßen Zusammensetzung. Hier hat sich gezeigt, dass ein solcher Anteil ausreichend ist, um die gewünschte Regulierung der Tröpfchengröße und damit ein angenehmes Hautgefühl beim Anwender zu erreichen.

Weiterhin wurde überraschend gefunden, dass auch ein gewisser Anteil an Wasser in der Zusammensetzung einen positiven Einfluss auf die Größe der Tröpfchen hat, die beim Versprühen der erfindungsgemäßen Zusammensetzung entstehen. Daher ist eine Ausführungsform bevorzugt, in der der Gehalt an Wasser in der erfindungsgemäßen Zusammensetzung 0,5 bis 15 Gew.-%, vorzugsweise 1 bis 5 Gew.-% beträgt, jeweils bezogen auf das Gesamtgewicht der Komponenten a) bis c) der erfindungsgemäßen Zusammensetzung.

Die besten Ergebnisse bezüglich Tröpfchengröße und Sprühbarkeit der erfindungsgemäßen Zusammensetzung wurden erreicht, wenn diese alkoholbasiert war. Daher beträgt der Gehalt an Ethanol in der erfindungsgemäßen Zusammensetzung, jeweils bezogen auf das Gesamtgewicht der Komponenten a) bis c) der Zusammensetzung vorzugsweise mindestens 70 Gew.-%, besonders bevorzugt mindestens 80 Gew.-%.

Der gewünschte Effekt betreffend die Tröpfchengröße wurde verstärkt in den Fällen beobachtet, in denen der eingesetzte Polyglycerinester eine Kettenlänge des Esters von bis zu 12 Kohlenstoffatomen aufwies. Daher ist eine Ausführungsform bevorzugt, in der der Polyglycerinester der erfindungsgemäßen Zusammensetzung einen Ester mit einer Kettenlänge von 8 bis 12 Kohlenstoffatomen umfasst. In einer besonders bevorzugten Ausführungsform handelt es sich bei dem Polyglycerinester um Polyglyceryl-3-Caprylat.

Die erfindungsgemäße Zusammensetzung ist insbesondere dafür ausgelegt, zur Bekämpfung von unangenehmen Körpergerüchen verwendet zu werden. Daher ist eine Ausführungsform bevorzugt, in die Zusammensetzung weiterhin Duftstoffe umfasst.

Die sprühbare, kosmetische Zusammensetzung der vorliegenden Erfindung lässt sich in üblicher Art und Weise herstellen. In der Regel werden alle Bestandteile der kosmetischen Zusammensetzung mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge des Treibmittels eingefüllt.

Zur Abgabe und Applikation der sprühbaren Zusammensetzung eigenen sich insbesondere Aerosolabgabebehälter. Ein weiterer Gegenstand der vorliegenden Erfindung ist daher ein kosmetisches Mittel umfassend eine Zusammensetzung gemäß der vorliegenden Erfindung und einen Aerosolabgabebehälter.

Unter der Bezeichnung Aerosolabgabebehälter sind Druckbehälter zu verstehen, in deren Inneren ein höherer Druck herrscht als außerhalb des Behälters und aus dem sich ein Gasstrom über ein Ventil entnehmen lässt. Bei den Aerosolabgabebehältern handelt es sich mit anderen Worten um Druckbehälter, mit deren Hilfe ein Produkt, zum Beispiel eine kosmetische Zusammensetzung, durch den inneren Gasdruck des Behälters über ein Ventil abgegeben werden kann.

Als Aerosolabgabebehälter kommen druckfeste Gefäße aus Metall, wie Aluminium, Weißblech oder Zinn), geschütztem beziehungsweise nicht-splitterndem Kunststoff oder Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit usw. eine Rolle spielen. Spezielle Innenschutzlacke gewährleisten die Korrosionsbeständigkeit gegenüber der im Druckbehälter konfektionierten Zusammensetzung. Besonders bevorzugt weisen die verwendeten Ventile einen innenlackierten Ventilteiler auf, wobei Lackierung und Ventilmaterial miteinander kompatibel sind. Werden Aluminiumventile eingesetzt, so können deren Ventilteiler innen zum Beispiel mit Microflex-Lack beschichtet sein. Werden erfindungsgemäß Weißblechventile eingesetzt, so können deren Ventilteiler zum Beispiel mit PET (Polyethylenterephthalat) beschichtet sein.

Gemäß der vorliegenden Erfindung können auch Mehrkammerspender als Aerosolabgabebehälter eingesetzt werden. Der Mehrkammspender kann so ausgestaltet sein, dass eine Kammer das komprimierte Treibmittel und eine andere Kammer mit den restlichen Bestandteilen der erfindungsgemäßen Zusammensetzung befüllt ist. Ein derartiger Mehrkammerspender ist beispielsweise eine so genannte Bag-in-Can-Verpackung.

Ein weiterer Gegenstand der vorliegenden Erfindung ist die Verwendung der erfindungsgemäßen sprühbaren, kosmetischen Zusammensetzung als Deodorant.

Die nachfolgenden Beispiele sollen den Gegenstand der vorliegenden Erfindung näher erläutern, ohne ihn in irgendeiner Weise einzuschränken.

Es wurden die folgenden Formulierungen hergestellt und in Aerosolabgabebehälter bis zu 50 bis 70% des Randvolumens abgefüllt und mit Stickstoff oder komprimierter Luft bis zu einem Druck von 7 bis 12 bar begast. Die Angaben sind jeweils in Gew.-%.

Tabelle 1:

| | E1 | E2 | E3 | E4 | E5 | E6 |
|---|---|---|---|---|---|---|
| Ethanol (96%ig) | 91,81 | 92,05 | 93,0 | 91,81 | 91,81 | 91,81 |
| Triethylcitrat | 6,0 | 6,0 | 6,0 | 6,0 | 6,0 | 6,0 |
| Phenoxyethanol | 0,75 | 0,75 | 0,75 | 0,75 | 0,75 | 0,75 |
| Polyglyceryl-3-Caprylat | 0,44 | 1,2 | 0,25 | 0,22 | - | - |
| Polyglyceryl-4-Laurat | - | - | - | - | 0,44 | - |
| Polyglyceryl-2-Caprat | - | - | - | 0,22 | - | 0,44 |

Die in Tabelle 2 zusammengefasst Formulierungen E1 und E7 bis E11 wurden in Aerosolabgabebehälter abgefüllt (60% des Randvolumens) und mit Stickstoff bis zu einem Druck von 9 bar begast. Die Behälter wurden abgesprüht bis nur noch 10% des ursprünglichen Inhalts vorhanden war. Dann wurde eine Partikelgrößenvermessung im Sprühstrahl mittels Laserbeugung (Spraytech, Malvern International Ltd.) vorgenommen. Der D(v)50-Wert gibt an, dass 50% des Probenvolumens in Partikeln vorliegt, die kleiner sind als der angegebene Wert.

Die Daten zeigen, dass insbesondere der Einsatz von Polyglyceryl-3-Caprylat zu einer deutlichen Verkleinerung der Tröpfchen im Sprühstrahl führt.

Die Angaben in der Tabelle beziehen sich jeweils auf Gew.-%.

Tabelle 2:

| | E1 | E7 | E8 | E9 | V |
|---|---|---|---|---|---|
| Ethanol (96%ig) | 91,81 | 91,81 | 91,81 | 91,81 | 92,25 |
| Triethylcitrat | 6,0 | 6,0 | 6,0 | 6,0 | 6,0 |
| Phenoxyethanol | 0,75 | 0,75 | 0,75 | 0,75 | 0,75 |
| Polyglyceryl-3-Caprylat (HLB 13) | 0,44 | - | - | - | - |
| Polyglyceryl-3-Oleat (HLB 9,5) | - | 0,44 | - | - | - |
| Polyglyceryl-10-Palmitat (HLB 15) | - | - | 0,44 | - | - |
| Polyglyceryl-10-Laurat (HLB 15,5) | - | - | - | 0,44 | - |
| Parfüm | 1,0 | 1,0 | 1,0 | 1,0 | 1,0 |
| **Tröpfchengröße** | | | | | |
| D(v)10 [$\mu$m] | 31 | 38 | 37 | 37 | 41 |
| D(v)50 [$\mu$m] | 62 | 69 | 69 | 68 | 75 |

**Patentansprüche**

1. Sprühbare, kosmetische Zusammensetzung umfassend:

    a) Ethanol;
    b) Polyglycerinester;
    c) Wasser; und
    d) Treibmittel.

2. Zusammensetzung gemäß Anspruch 1, **dadurch gekennzeichnet, dass** der Polyglycerinester einen HLB-Wert von 9 bis 15, vorzugsweise 11 bis 14 aufweist, bestimmt gemäß Molekulargewichtsberechnung.

3. Zusammensetzung gemäß wenigstens einem der Ansprüche 1 oder 2, **dadurch gekennzeichnet, dass** als Treibmittel ein komprimiertes Gas, vorzugsweise ein Gas ausgewählt aus der Gruppe bestehend aus Luft, Stickstoffoxid, Stickstoff, Kohlenstoffdioxid und Mischungen davon, insbesondere Stickstoff, verwendet wird.

4.  Zusammensetzung gemäß wenigstens einem der vorangehenden Ansprüche, **dadurch gekennzeichnet, dass** der Gehalt an Polyglycerinester bezogen auf das Gesamtgewicht der Komponenten a) bis c) der Zusammensetzung 0,05 bis 5 Gew.-%, vorzugsweise 0,2 bis 0,6 Gew.-% beträgt.

5.  Zusammensetzung gemäß wenigstens einem der vorangehenden Ansprüche, **dadurch gekennzeichnet, dass** der Gehalt an Wasser, bezogen auf das Gesamtgewicht der Komponenten a) bis c) der Zusammensetzung 0,5 bis 15 Gew.-%, vorzugsweise 1 bis 5 Gew.-% beträgt.

6.  Zusammensetzung gemäß wenigstens einem der vorangehenden Ansprüche, **dadurch gekennzeichnet, dass** der Gehalt an Ethanol, bezogen auch das Gesamtgewicht der Komponenten a) bis c) der Zusammensetzung mindestens 70 Gew.-%, vorzugsweise mindestes 80 Gew.-% beträgt.

7.  Zusammensetzung gemäß wenigstens einem der vorangehenden Ansprüche, **dadurch gekennzeichnet, dass** es sich bei dem Polyglycerinester um Polyglyceryl-3-Caprylat handelt.

8.  Zusammensetzung gemäß wenigstens einem der vorangehenden Ansprüche, **dadurch gekennzeichnet, dass** die Zusammensetzung weiterhin Duftstoffe umfasst.

9.  Kosmetisches Mittel umfassend eine Zusammensetzung gemäß wenigstens einem der Ansprüche 1 bis 8 und einen Aerosolabgabebehälter.

10. Verwendung einer kosmetischen Zusammensetzung gemäß wenigstens einem der Ansprüche 1 bis 8 als Deodorant.

EUROPÄISCHER RECHERCHENBERICHT

Nummer der Anmeldung

EP 22 16 0692

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2015 210481 A1 (HENKEL AG & CO KGAA [DE]) 15. Dezember 2016 (2016-12-15) * Formulierung 2; Absätze [0035], [0053], [0060]; Ansprüche 1,10; Beispiele * ----- | 1,2,4-10 | INV. A61K8/04 A61K8/19 A61K8/37 A61K8/39 A61K8/34 A61Q15/00 |
| X | WO 2016/098045 A1 (ARTSANA SPA [IT]) 23. Juni 2016 (2016-06-23) * Seite 11 * ----- | 1,2,4, 6-10 | |
| X | DATABASE GNPD [Online] MINTEL; 1. Juli 2008 (2008-07-01), anonymous: "Deodorant for Men", XP055962429, Database accession no. 937879 * Zusammenfassung * ----- | 1-3,7-10 | |
| X | DATABASE GNPD [Online] MINTEL; 12. April 2017 (2017-04-12), anonymous: "Cologne Body Spray", XP055962132, Database accession no. 4751565 * Zusammenfassung * ----- | 1-3,7-10 | RECHERCHIERTE SACHGEBIETE (IPC) A61K A61Q |
| A | US 10 959 929 B2 (HENKEL AG & CO KGAA [DE]) 30. März 2021 (2021-03-30) * Zusammenfassung; Ansprüche; Beispiele * * Spalte 10, Zeile 22 – Zeile 35 * ----- -/-- | 1-10 | |

Der vorliegende Recherchenbericht wurde für alle Patentansprüche erstellt

| Recherchenort | Abschlußdatum der Recherche | Prüfer |
|---|---|---|
| München | 21. September 2022 | Blott, Catherine |

EPO FORM 1503 03.82 (P04C03)

EUROPÄISCHER RECHERCHENBERICHT

Nummer der Anmeldung

EP 22 16 0692

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | Anonymous: "Glycerol polymerization degree effect on the emulsifying properties of polyglycerol esters – Díaz-Lasprilla – 2021 – Journal of Applied Polymer Science – Wiley Online Library", , 11. Februar 2021 (2021-02-11), XP055962448, Gefunden im Internet: URL:https://onlinelibrary.wiley.com/doi/full/10.1002/app.50566 [gefunden am 2022-09-19] * das ganze Dokument * ----- | 1-10 | |
| A | DATABASE CA [Online] CHEMICAL ABSTRACTS SERVICE, COLUMBUS, OHIO, US; DOLINNYI, A. I. ET AL: "Phase changes in surfactant solutions of different natures", XP002807556, gefunden im STN Database accession no. 107:103242 * Zusammenfassung * & DOLINNYI, A. I. ET AL: "Phase changes in surfactant solutions of different natures", OTKHODY PROM-STI. MINER. SYR'E PROIZVOD. TEKH. STROIT. MATER. ( LENINGR. OTD, LENINGRAD, USSR. CODEN: 55VPAC, 1986, ----- | 1-10 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 21. September 2022 | Blott, Catherine |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

# EP 4 088 711 A1

## ANHANG ZUM EUROPÄISCHEN RECHERCHENBERICHT
## ÜBER DIE EUROPÄISCHE PATENTANMELDUNG NR.

EP 22 16 0692

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

21-09-2022

| Im Recherchenbericht angeführtes Patentdokument | Datum der Veröffentlichung | Mitglied(er) der Patentfamilie | Datum der Veröffentlichung |
|---|---|---|---|
| DE 102015210481 A1 | 15-12-2016 | DE 102015210481 A1<br>EP 3307232 A1<br>US 2018140522 A1<br>WO 2016198202 A1 | 15-12-2016<br>18-04-2018<br>24-05-2018<br>15-12-2016 |
| WO 2016098045 A1 | 23-06-2016 | KEINE | |
| US 10959929 B2 | 30-03-2021 | DE 102017217735 A1<br>FR 3072030 A1<br>GB 2568812 A<br>US 2019105251 A1 | 11-04-2019<br>12-04-2019<br>29-05-2019<br>11-04-2019 |

EPO FORM P0461

Für nähere Einzelheiten zu diesem Anhang : siehe Amtsblatt des Europäischen Patentamts, Nr.12/82

8